# EUROPEAN PATENT APPLICATION

(11) **EP 4 420 658 A1**
(43) Date of publication of application: **28.08.2024**
(21) Application number: 24159528.9
(22) Date of filing: 23.02.2024
(51) Int. Cl.: A61K 9/20, A61K 9/28, A61K 31/7048

(54) **A FILM COATED TABLET COMPRISING EMPAGLIFLOZIN**

(30) Priority: 24.02.2023 TR 202302079
(71) Applicant: SANOVEL ILAC SANAYI VE TICARET A.S., Istanbul 34460 (TR)
(72) Inventor: SUNEL, Fatih, Istanbul (TR); PEHLIVAN AKALIN, Nur, Istanbul (TR); ARMUT, Merve, Istanbul (TR)
(74) Representative: Sevinç, Erkan

(57) **Abstract**

The present invention relates to a film coated tablet comprising empagliflozin or a pharmaceutically acceptable salt thereof or crystalline polymorph thereof and at least one pharmaceutically acceptable excipient wherein used lubricant is sodium stearyl fumarate. The present invention also relates to a simple, rapid, cost effective, time-saving and industrially convenient method of preparing the formulation.

## Description

### Field of the Invention

The present invention relates to a film coated tablet comprising empagliflozin or a pharmaceutically acceptable salt thereof or crystalline polymorph thereof and at least one pharmaceutically acceptable excipient wherein used lubricant is sodium stearyl fumarate. The present invention also relates to a simple, rapid, cost effective, time-saving and industrially convenient method of preparing the formulation.

### Background of the Invention

Empagliflozin is a potent inhibitor of sodium-glucose co-transporter type 2 (SGLT-2) and is therefore used to treat type 2 diabetes. It is currently approved for the treatment of type 2 diabetes and improvement of blood sugar control.

Empagliflozin is a white to yellowish non-hygroscopic crystalline solid, very slightly soluble in water (pH 1-7.4), slightly soluble in acetonitrile and ethanol, sparingly soluble in methanol, and practically insoluble in toluene. The chemical name of empagliflozin (1S)-1,5-anhydro-1-(4-chloro-3-{4-[(3S)-tetrahydrofuran-3-yloxy]benzyl}phenyl)-D- glucitol and its chemical structure is shown in the Formula- I.

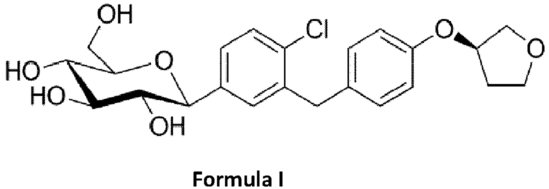

Empagliflozin is available on the market in the form of a free base and is sold under trade name Jardiance<^{®}>, as an oral tablet in 10 mg and 25 mg strengths. Further it is available on the market as a combination product with Metformin and a combination product with Linagliptin.

The first mention of the crystalline form of empagliflozin can be found in patent application WO 2006/117359. WO2006117360 discloses two other forms of empagliflozin which is described as Form I and Form II. Also, it disclosed methods for production thereof and synthesis of Empagliflozin.

US 20110014284 describes pharmaceutical compositions comprising empagliflozin, process for the preparation of such compositions and their use in the treatment of various conditions including type 1 diabetes mellitus and type 2 diabetes mellitus.

EP1730131 discloses Empagliflozin, process for production thereof, its use and pharmaceutical composition thereof. The composition of a tablet is disclosed that comprises active ingredient in admixture with pharmaceutically acceptable excipients, such as lactose, com starch, polyvinylpyrrolidone, magnesium stearate.

As evident from the prior art and the literature there are reported several pharmaceutical compositions comprising Empagliflozin. There is an existing and continual need for oral dosage formulations comprising Empagliflozin or a pharmaceutically acceptable salt thereof that have high stability, content uniformity, improved compressibility, flowability and manufactured by safe, effective, easy manufacturing methods.

We have found that the film coated tablet comprising empagliflozin or a pharmaceutically acceptable salt thereof and sodium stearyl fumarate as lubricant, it positively affects the desired high stability. The obtained tablets have also the desired compressibility and homogeneous.

### Detailed Description of the Invention

The main object of the present invention is to provide a film coated tablet comprising a therapeutically effective amount of Empagliflozin or a pharmaceutically acceptable salt thereof or crystalline polymorph thereof with the desired high stability.

Another object of the present invention is to provide a film coated tablet comprising Empagliflozin or a pharmaceutically acceptable salt thereof or crystalline polymorph thereof with improved compressibility, flowability, homogeneity and the desired dissolution profile.

Another object of the present invention is to provide a film coated tablet comprising Empagliflozin or a pharmaceutically acceptable salt thereof or crystalline polymorph thereof prepared by simple, easy, time-saving and fast manufacturing methods.

As it is known, the use of lubricant provides excellent flowability of powders during process comprising empagliflozin. We have also seen in the present invention that the use of sodium stearyl fumarate shows good stability to the tablet.

According to one embodiment of this invention, a film coated tablet comprises empagliflozin or a pharmaceutically acceptable salt thereof or crystalline polymorph thereof and at least one pharmaceutically acceptable excipient wherein used lubricant is sodium stearyl fumarate.

According to one embodiment of this invention, the amount of sodium stearyl fumarate is between 0.1% and 3.0% or 0.1% and 1.3% by weight in the total core tablet.

According to one embodiment of the present invention, the amount of Empagliflozin is present between 0.5% and %25.0 by weight in the total composition. Preferably, the amount of Empagliflozin is between 1.0% and 20.0% or 3.0% and 14.0% by weight in the total core tablet.

According to one embodiment of the present invention, preferably, empagliflozin is in the form of crystalline form.

In general terms, excipients provided in a formulation may positively or negatively influence the physicochemical and pharmacokinetic properties, e.g. the solubility, absorption, bioavailability of an active agent. For this reason, the excipients which accompany an active agent have to be selected in a careful and conscious manner while a formulation is developed. The formulations should have no physicochemical incompatibility between the active agent and the excipients.

According to this embodiment of the present invention, the film coated tablet comprises at least one pharmaceutically acceptable excipient is selected from the group comprising fillers, binders, disintegrants, glidants or mixtures thereof.

Suitable fillers are selected from the group comprising lactose, lactose monohydrate, spray-dried lactose monohydrate, anhydrous lactose, microcrystalline cellulose, pregelatinized starch, mannitol, corn starch, maltodextrin, fructose, maltose, sucrose, dicalcium phosphate, ammonium alginate, calcium carbonate, calcium phosphate, calcium phosphate dehydrate, neutral pellets, cellulose, cellulose acetate, magnesium oxide, maltodextrin, medium chain triglycerides, polydextrose, polymethacrylates, sodium alginate, starch, sugar sphericals, polysorbate 80 or mixtures thereof.

According to an embodiment of the present invention, the amount of fillers are 50.0% to 95.0% by weight in the total core tablet. Preferably, it is between 70.0% to 92.0% or between 75.0% and 90.0% by weight in the total core tablet. The use of high amount filler has a good effect both for the dissolution profile and for powder homogeneity.

According to one embodiment of the present invention, the filler is microcrystalline cellulose.

According to one embodiment of the present invention, the filler is lactose monohydrate.

According to one embodiment of the present invention, the fillers are microcrystalline cellulose and lactose monohydrate.

According to this embodiment of the present invention, the film coated tablet comprises;
- Empagliflozin crystalline form
- Microcrystalline Cellulose
- Lactose monohydrate
- Sodium stearyl fumarate. The tablet has the desired stability and the desired dissolution profile.

Suitable binders are selected from the group comprising corn starch, hydroxypropyl cellulose, carboxymethylcellulose sodium, starch mucilage, acacia mucilage, dextrose, ethyl cellulose, glyceryl behenate, hydroxyethyl cellulose, hydroxyethyl methyl cellulose, hydroxypropyl starch, hypromellose, magnesium aluminum silicate, maltodextrin, maltose, methylcellulose, poloxamer, polydextrose, polyethylene oxide, polymethacrylates or mixtures thereof.

According to this embodiment of the present invention, the amount of binders is between 1.0% and 10.0% by weight of the total core tablet. Preferably, the amount of binders is between 1.0% and 4.0% by weight of the total core tablet.

According to one embodiment of the present invention, the ratio of sodium stearyl fumarate to binder is in the range of 1:5 to 1:7. This ratio provides the desired compressibility and flowability.

According to this embodiment of the present invention, the binder is hydroxypropyl cellulose. Especially, hydroxypropyl cellulose LF is used.

Suitable disintegrants are selected from the group comprising sodium starch glycolate, croscarmellose sodium, crospovidone, magnesium aluminum silica, sodium carboxymethyl cellulose, calcium silicate, carboxymethyl cellulose, calcium carboxymethyl cellulose, low substituted hydroxypropyl cellulose, poloxamer, sodium alginate, sodium glycine carbonate, sodium dodecyl sulfate or mixtures thereof.

According to this embodiment of the present invention, the amount of disintegrants is between 1.0% and 10.0% by weight of the total core tablet. Preferably, the amount of disintegrants is between 1.0% and 4.0% by weight of the total core tablet.

According to this embodiment of the invention, the disintegrant is croscarmellose sodium.

Suitable glidants are selected from the group comprising anhydrous colloidal silicon dioxide, calcium stearate, talc, sodium chlorate, sodium benzoate, polyethylene glycol, sodium lauryl sulfate, aluminum silicate, colloidal silica, calcium silicate, magnesium silicate, magnesium oxide, starch or mixtures thereof.

According to this embodiment of the invention, the glidant is anhydrous colloidal silicon dioxide.

According to an embodiment of the present invention, the pharmaceutical formulation comprises;
a) Empagliflozin
b) Microcrystalline Cellulose
c) Lactose monohydrate
d) HPC LF
e) Croscarmellose sodium
f) Anhydrous colloidal silicon dioxide
g) Sodium stearyl fumarate

According to one embodiment of the present invention, the film coated tablet comprising empagliflozin or a pharmaceutically acceptable salt thereof is obtained by wet granulation process. This process provides homogeneity.

According to one embodiment of the present invention, a process for the preparation of the film coated tablet comprises;
- Mixing Empagliflozin, microcrystalline cellulose, lactose monohydrate and HPC,
- Granulating the mixture with water,
- Sieving the wet granule,
- Drying the wet granule in a fluidized bed,
- Sieving the mixture,
- Adding croscarmellose sodium and anhydrous colloidal silicon dioxide and then mixing,
- Adding sodium stearyl fumarate and then mixing,
- Compressing the mixture into the tablet,
- Coating the tablets with film coating agent.

### Example 1: Film coated tablet comprising empagliflozin

| **Ingredients** | **Amount (% by weight of the total core tablet)** |
|---|---|
| Empagliflozin | 0.5 - 25.0 |
| Microcrystalline Cellulose PH101 | 20.0 - 27.0 |
| Lactose Monohydrate | 50.0 - 68.0 |
| HPC LF | 1.0 - 10.0 |
| Croscarmellose sodium | 1.0 - 10.0 |
| Anhydrous colloidal silicon dioxide | 0.1 - 3.0 |
| Sodium stearyl fumarate | 0.1 - 3.0 |
| **TOTAL CORE TABLET** | **100** |

### Example 2: Film coated tablet comprising empagliflozin

| **Ingredients** | **Amount (% by weight of the total core tablet)** | **Amount (% by weight of the total core tablet)** |
|---|---|---|
| Empagliflozin | 12.50 | 4.0 |
| Microcrystalline Cellulose PH101 | 25.00 | 25.00 |
| Lactose Monohydrate | 56.50 | 65.0 |
| HPC LF | 3.00 | 3.00 |
| Croscarmellose sodium | 2.00 | 2.00 |
| Anhydrous colloidal silicon dioxide | 0.50 | 0.50 |
| Sodium stearyl fumarate | 0.50 | 0.50 |
| **TOTAL CORE TABLET** | **100** | **100** |

A process for example 1 or 2;
- Mixing Empagliflozin, microcrystalline cellulose, lactose monohydrate and HPC,
- Granulating the mixture with water,
- Sieving the wet granule,
- Drying the wet granule in a fluidized bed,
- Sieving the mixture,
- Adding croscarmellose sodium and anhydrous colloidal silicon dioxide and then mixing,
- Adding sodium stearyl fumarate and then mixing,
- Compressing the mixture into the tablet,
- Coating the tablets with film coating agent.

### Film coating

| |
|---|
| *Hypromellose* |
| *Titanium Dioxide (E171)* |
| *Talc* |
| *Macrogol (400)* |
| *Iron oxide yellow (E172)* |

## Claims

1. A film coated tablet comprises empagliflozin or a pharmaceutically acceptable salt thereof or crystalline polymorph thereof and at least one pharmaceutically acceptable excipient wherein used lubricant is sodium stearyl fumarate.

2. The film coated tablet according to claim 1, wherein the amount of sodium stearyl fumarate is between 0.1% and 3.0% or 0.1% and 1.3% by weight in the total core tablet.

3. The film coated tablet according to claim 1, wherein empagliflozin is in the form of crystalline form.

4. The film coated tablet according to claim 1, wherein the film coated tablet comprises at least one pharmaceutically acceptable excipient is selected from the group comprising fillers, binders, disintegrants, glidants or mixtures thereof.

5. The film coated tablet according to claim 4, wherein fillers are selected from the group comprising lactose, lactose monohydrate, spray-dried lactose monohydrate, anhydrous lactose, microcrystalline cellulose, pregelatinized starch, mannitol, corn starch, maltodextrin, fructose, maltose, sucrose, dicalcium phosphate, ammonium alginate, calcium carbonate, calcium phosphate, calcium phosphate dehydrate, neutral pellets, cellulose, cellulose acetate, magnesium oxide, maltodextrin, medium chain triglycerides, polydextrose, polymethacrylates, sodium alginate, starch, sugar sphericals, polysorbate 80 or mixtures thereof.

6. The film coated tablet according to claim 5, wherein the amount of fillers is 50.0% to 95.0% by weight in the total core tablet.

7. The film coated tablet according to claim 5, wherein the filler is microcrystalline cellulose.

8. The film coated tablet according to claim 5, wherein the filler is lactose monohydrate.

9. The film coated tablet according to claim 3 or 5, wherein comprising;
- Empagliflozin crystalline form
- Microcrystalline Cellulose
- Lactose monohydrate
- Sodium stearyl fumarate.

10. The film coated tablet according to claim 4, wherein binders are selected from the group comprising corn starch, hydroxypropyl cellulose, carboxymethylcellulose sodium, starch mucilage, acacia mucilage, dextrose, ethyl cellulose, glyceryl behenate, hydroxyethyl cellulose, hydroxyethyl methyl cellulose, hydroxypropyl starch, hypromellose, magnesium aluminum silicate, maltodextrin, maltose, methylcellulose, poloxamer, polydextrose, polyethylene oxide, polymethacrylates or mixtures thereof.

11. The film coated tablet according to claim 2 or 10, wherein the ratio of sodium stearyl fumarate to binder is in the range of 1:5 to 1:7.

12. The film coated tablet according to claim 10, wherein binder is hydroxypropyl cellulose.

13. The film coated tablet according to claim 4, wherein disintegrants are selected from the group comprising sodium starch glycolate, croscarmellose sodium, crospovidone, magnesium aluminum silica, sodium carboxymethyl cellulose, calcium silicate, carboxymethyl cellulose, calcium carboxymethyl cellulose, low substituted hydroxypropyl cellulose, poloxamer, sodium alginate, sodium glycine carbonate, sodium dodecyl sulfate or mixtures thereof.

14. The film coated tablet according to claim 4, wherein glidants are selected from the group comprising anhydrous colloidal silicon dioxide, calcium stearate, talc, sodium chlorate, sodium benzoate, polyethylene glycol, sodium lauryl sulfate, aluminum silicate, colloidal silica, calcium silicate, magnesium silicate, magnesium oxide, starch or mixtures thereof.

15. The film coated tablet according to claim 1, wherein the pharmaceutical formulation comprises;
a) Empagliflozin
b) Microcrystalline Cellulose
c) Lactose monohydrate
d) HPC LF
e) Croscarmellose sodium
f) Anhydrous colloidal silicon dioxide
g) Sodium stearyl fumarate
